Europäisches Patentamt

(19) European Patent Office

Office européen des brevets

(11) Publication number: **0 139 595**
**A2**

## (12) EUROPEAN PATENT APPLICATION

(21) Application number: **84402110.5**

(22) Date of filing: **19.10.84**

(51) Int. Cl.⁴: **A 23 K 1/17**
**A 23 K 1/18**

(30) Priority: **20.10.83 IL 70015**

(43) Date of publication of application:
**02.05.85 Bulletin 85/18**

(84) Designated Contracting States:
**AT BE CH DE FR GB IT LI LU NL SE**

(71) Applicant: **KOFFOLK (1949) LTD.**
**Industrial area Kiryat Matalon, Petach Tikvah P.O. Box 1098**
**Tel Aviv(IL)**

(72) Inventor: **Niv, Arieh**

**Ariel 105 2c(IL)**

(74) Representative: **Santarelli, Marc et al,**
**Cabinet Rinuy et Santarelli 14, avenue de la Grande Armée**
**F-75017 Paris(FR)**

(54) **Ionophore antibiotic compositions for ruminants and poultry.**

(57) The invention provides a liquid ionophore antibiotic composition for ruminants and poultry.

The ionophore antibiotic is dissolved in a non-toxic water-soluble organic solvent and the resulting solution is admixed with a liquid feed, a liquid vitamin concentrate, or drinking water.

These compositions remain substantially uniform on standing even for long periods.

EP 0 139 595 A2

Croydon Printing Company Ltd.

The present invention relates to ionophore antibiotic compositions for ruminants and poultry and to processes for the production thereof.

More particularly, the present invention relates to liquid feeds and milk replacers for ruminants, particularly for suckling ruminants, water-soluble vitamin concentrates as well as drinking water·compositions for same, and for poultry, said compositions or concentrates containing ionophore antibiotics.

It is known that ionophore antibiotics such as monensin, narasin, lasalocid and solinomycin, when added to feed mixtures for ruminants increase feed-conversion and rate of growth in addition to the anticoccidial effect of these antibiotics.

While such ionophore antibiotics as monensin have been known since 1967 for their therapeutic veterinary effect as antiprotozoal, antibacterial and antifungal agents as well as for their coccidiostatic effect in poultry, as reported, e.g., by Ilan et al Anim. Prod. 1981, 32:125-131, because of the fact that monensin and its sodium salt are only slightly soluble in water, they are· generally administered in dry form in an animal feed and/or in dry liquid-milk replacer compositions.

The same is true for lasalocid which is described and claimed in German Pat. 2,040,998 and in corresponding U.S. Patent 3,715,372 and which is reported to be completely insoluble in water.

Thus while said dry form of administration has been available and used for more than a decade it is known and recognized that young and/or sick livestock cannot always be induced to eat and thus despite the long-felt need and desire in the industry to find an alternate means of administration of said antibodies, to date no such method or product has been suggested or proposed.

On occasion and in desperation, products such as monesin have been vigorously mixed with livestock liquid feeds in the hope that the livestock in drinking such feeds would ingest sufficient ionophore product to meet its needs however in fact what was observed to occur was that as a result of the insolubility of said ionophore antibiotics gradients of antibiotic concentrations are formed in the compositions on standing, such that the top layers contain ineffectially low concentrations of the antibiotics, while the bottom layers contain the antibiotics in toxic, harmful concentrations causing poisoning of the animals fed with these compositions.

Thus even in the latest FDA Status Form FDA 1800 (1983) appears the limitation for liquid feed administration of monensin that any liquid feed containing monensin must be agitated prior to use, thus again indicating the recognized instability of monensin distribution in solution and the problems inherent in its use in other than dry form, which heretofor has been dealt with by requiring vigorous mixing immediately prior to administration to assure ingestion before substantial concentration gradients begin to appear due to settling.

It has now been found that ionophore antibiotics can be provided in non-toxic organic solvents which are fully miscible with aqueous solutions of feed-or vitamin compositions. The resulting mixtures remain substantially uniform on standing even for long periods.

This invention thus relates more particularly to a liquid ionophore antibiotic composition for ruminants and poultry comprising an ionophore antibiotic and at least one non-toxic water-miscible organic solvent.

Preferably said antibiotic is selected from the group including monensin, lasalocid, narasin and solinomycin and monensin is especially preferred.

The organic solvents used are preferably selected from the group including propylene glycol, glycerol, ethanol and isopropanol and from mixtures of these solvents.

The invention also provides a dry water-soluble ionophore antibiotic composition suitable for combination with an aqueous medium to form a liquid composition for ruminants and poultry comprising an ionophore antibiotic; a non-toxic, water-miscible, organic solvent and a dry, water-soluble carrier therefor.

Preferably said dry water-soluble carrier is a dry animal feed or a dry milk replacer adapted to be dissolved in a liquid medium before administration.

The invention furthermore relates to a process for preparing liquid feeds, liquid vitamin concentrates and drinking water containing ionophore antibiotics for ruminants and for poultry respectively, said process comprising dissolving an ionophore antibiotic in a non-toxic water soluble organic solvent and admixing the resulting solution with a liquid feed, a water soluble vitamin concentrate or with drinking water.

In said process the non-toxic organic solutions of the ionophore antibiotics are preferably obtained by extraction of the mycelia of the respective antibiotics with the non-toxic organic solvents followed by separation by centrifuge.

Said liquid feed is preferably a liquid milk replacer and said vitamin concentrate is preferably a vitamin A+D concentrate, although in light of the teachings of the present invention the ionophore antibiotic compositions of the present invention can be incorporated in any liquid administered to ruminants or poultry as well as in any water-soluble dry preparation sold for later combination with aqueous medium.

Thus the invention also provides a process for preparing liquid feeds containing ionophore antibiotics comprising dissolving an ionophore antibiotic in a non-toxic water soluble organic solvent, spraying the resulting liquid on a dry water soluble animal feed and subsequently combining the resulting dry antibiotic-containing feed with liquid.

As will be realized the present invention now also provides a method of increasing feed conversion and growth-rate of suckling ruminants and poultry respectively, wherein solutions of ionophore antibiotics in water-soluble non-toxic organic solvents incorporated in liquid feeds, milk replacers, water-soluble vitamin concentrates or drinking water are administered to the animals.

While the invention will now be described in connection with certain preferred embodiments in the following examples, it will be understood that it is not intended to limit the invention to these particular embodiments. On the contrary, it is intended to cover all alternatives, modifications and equivalents as may be included within the scope of the invention as defined by the appended claims. Thus the following examples which include preferred embodiments will serve to illustrate the practice of this invention, it being understood that the particulars described are by way of example and for purposes of illustrative discussion of preferred embodiments of the present invention only and are presented in the cause of providing what is believed to be the most useful and readily understood description of procedures as well as of the principles and conceptual aspects of the invention.

A.  Preparation of the ionophore antibiotic solutions:

## EXAMPLE 1

250 g of a mycelium containing 10% monensin were mixed at room temperature with 1250 cc propylene glycol for 2 hours by exposing the mixture to ultrasound.  50% of the monensin was found in solution in the propylene glycol.  The undissolved monensin containing mycelium was separated from the monensin solution by centrifuge.

## EXAMPLE 2

500 g of a mycelium containing 10% monensin was mixed in a shaker with 1250 cc of propylene glycol at 50°C for 60 minutes.  40% of the monensin was found in the solution and separated from the solution by centrifuge.

## EXAMPLE 3

A mycelium containing 10% of monensin was sieved and the fraction passing through 60 mesh (250 microns) was extracted with 400 ml of ethanol by shaking for 3 hours at 45°C.  Almost 100% of the monensin was found in the alcoholic fraction which was separated by centrifuge.

## EXAMPLE 4

A concentrate of 15% lasalocid ("Avatec"$^{(R)}$ HLR) was extracted with ethanol at ratio of 100 g lasalocid concentrate with 300 ml ethanol for 3 hours in a mechanical shaker at 45°C.  Almost 13.5 g of the lasalocid was found in the alcoholic fraction which was separated by centrifuge.

## EXAMPLE 5

100 g of 15% lasalocid was extracted by shaking with 500 ml propylene glycol for 3 hours at 45°C.   About 60% of the drug was
found in the solution after separation by centrifuge.


EXAMPLE 6

The mycelium containing 10% monensin was concentrated to 16%
by sieving through a 250 micron sieve.   100 g of this concentrated
material was extracted with 400 ml of a mixture containing 60%
propylene glycol and 40% ethanol by mixing for 1 hour at room
temperature with a magnetic stirrer.   Almost all the monensin was
found in the solution which was separated by centrifuge.


EXAMPLE 7

Monensin mycelium was concentrated by passing through a 70
mesh (210 microns) sieve to almost 20%.   200 g of this material was
mixed in a turbomixer with 800 ml glycerol.   About 80% of the
monensin was found in the supernatant liquid; after centrifuging
almost 75% of the monensin was left in the clear liquid (conc. 36).
This was then mixed again with a liquid vitamin A+D concentrate
in molasses at a theoretical concentration of 0.72%.   The analysis
of this material gave 0.73% and 0.72% both on top and bottom of
this material after 7 days storage.

Direct addition of the glycerol solution to water at theoretical
level of 360 ppm (1°100) gave the following results after 7 days
storage:   400 ppm on bottom, 250 ppm on top.


EXAMPLE 8

A solution of 4% monensin in a mixutre of 60% propylene glycol
and 40% ethanol was prepared by extracting a mycelium containing
20% monensin with a corresponding mixture of these solvents.   The
solution of monensin was separated by centrifuge.


B.   Preparation of milk replacers, vitamin concentrates and drinking
water compositions from the above prepared solutions.

EXAMPLE 9

A liquid vitamin A+D concentrate in molasses (A-D-Vit) was
mixed with the monensin solution obtained in accordance with Example 1.

The mixture obtained was either added to water and administered to calves as drinking water or it was directly fed to them.

## EXAMPLE 10

The monensin solution obtained according to Example 6 was mixed with a liquid vitamin A+D preparation in molasses at a ratio 4.5 to 1. This mixture was then dissolved in water to yield a theoretical monensin level of 250 ppm. The composition obtained was left standing for 2 hours and the top and bottom layers of the compositions obtained in the container were analysed individually.
Result:   Concentration of monensin on top 180 ppm
Concentration of monsenin at the bottom 260 ppm

## EXAMPLE 11

The monensin solution obtained in accordance with Example 7 was admixed with liquid vitamin A+D concentrate in molasses to a theoretical monensin concentration of 0.72%. Separate analysis of the top and bottom layer monensin concentrations of the composition after 7 days storage yielded the following results:
Top layer 0.72%
Bottom layer 0.73%

## EXAMPLE 12

2 g of a 4% monensin solution obtained in accordance with Example 8 was admixed with 5 liters of milk replacer, mixed well and then analyzed microbiologically after leaving it standing for 30 minutes. The following results were obtained:
Monensin concentration at the top layer        13 ppm
Monensin concentration at the middle layer       17 ppm
Monensin concentration at the bottom layer       14 ppm
The theoretical monensin concentration in the mixture was 16 ppm. The results obtained in Examples 10 to 12 show that the ionophore antibiotic concentration of compositions containing the organic solvent solutions hereinbefore described remain substantially uniform on storage.

## EXAMPLE 13

80 g of a 4% monensin solution as obtained in Example 8 was sprayed in a horizontal mixer on 10 kg dry milk replacer. The dry milk replacer thus treated was subsequently dissolved with water according to standard preparation proceedings to form a 12.5% dry matter solution. Samples of the resultant solution were analyzed for monensin after standing for one hour. The following results were obtained:

Monensin concentration at the top layer        38 ppm
Monensin concentration at the middle layer     41 ppm
Monensin concentration at the bottom layer     44 ppm

## EXAMPLE 14

A lasalocid solution obtained according to Example 4 and containing 4.8% lasalocid in ethanol was diluted with propylene glycol in a ratio of 1: 50 (48% lasalocid in ethanol : propylene glycol) giving a solution of 0.095% lasalocid.

## EXAMPLE 15

A lasalocid 0.095% solution prepared according to Example 14 was mixed with water at a ratio of 1 to 20. After 2 hours standing the upper, middle and bottom layers were analyzed with the following results:

Top Layer        43 ppm
Middle Layer     42 ppm
Bottom Layer     44 ppm

## EXAMPLE 16

A 50 gm sample of 10% Narasin premix (Monteban [R] ELY) was extracted with 150 gm of ethanol for 2 hours in a mechanical shaker at room temperature. 4.69 gm of narasin was recovered in the alcoholic fraction which was separated by centrifuge.

## EXAMPLE 17

5 grams of the extract obtained in accordance with Example 16 (3.3%) was mixed with 80 grams of propylene glycol giving a solution of 0.21% Narasin.

## EXAMPLE 18

1 gm of the solution obtained according to Example 17 was mixed in 96 grams of water in separate analyses of the top and bottom layers. After 4 hours standing the following results were found:

| | |
|---|---|
| Top Layer | 21 ppm Narasin |
| Lower Layer | 23 ppm Narasin |

It will be evident to those skilled in the art that the invention is not limited to the details of the foregoing illustrative embodiments and examples and that the present invention may be embodied in other specific forms without departing from the essential attributes thereof, and it is, therefore, desired that the present embodiments and examples be considered in all respects as illustrative and not restrictive, reference being made to the appended claims, rather than to the foregoing description, and all changes which come with the meaning and range of equivalency of the claims are, therefore, intended to be embraced therein.

WHAT IS CLAIMED IS:

1. A liquid ionophore antibiotic composition for ruminants and poultry comprising an ionophore antibiotic and at least one non-toxic water-miscible organic solvent, said antibiotic being for instance selected from the group including monensin, lasalocid, narasin and solinomycin and said organic solvent being for instance selected from the group including propyleneglycol, glycerol, ethanol, isopropanol and mixtures thereof.

2. A liquid ionophore antibiotic composition for ruminants and poultry according to claim 1 in admixture with an aqueous solution.

3. A process for preparing liquid feeds, liquid vitamin concentrates and drinking water containing ionophore antibiotics for ruminants and for poultry respectively, said process comprising dissolving an ionophore antibiotic in a non-toxic water-soluble organic solvent and admixing the resulting solution with a liquid feed, a water soluble vitamin concentrate or with drinking water, said antibiotic being for instance selected from monensin, lasalocid, narasin or solinomycin and said organic solvent being for instance selected from the group including propyleneglycol, glycerol, ethanol, isopropanol and mixtures thereof.

4. A process as claimed in claim 3 wherein said liquid feed is a liquid milk replacer or wherein the vitamin concentrate is a vitamin A+D concentrate.

5. A process as claimed in claim 3, wherein the non-toxic organic solutions of the ionophore antibiotics are obtained by extraction of the mycelia of the respective antibiotics with the non-toxic organic solvents followed by separation by centrifuge.

6. A process for preparing liquid feeds containing ionophore antibiotics comprising dissolving an ionophore antibiotic in a non-toxic water soluble organic solvent, spraying the resulting liquid on a dry water soluble animal feed and subsequently combining the resulting dry antibiotic-containing feed with liquid.

7. A dry water soluble ionophore antibiotic composition suitable for combination with an aqueous medium to form a liquid composition for ruminants and poultry comprising an ionophore antibiotic; a non-toxic, water-miscible, organic solvent and a dry, water-soluble carrier therefor, said carrier being preferably a dry animal feed and more preferably a dry milk replacer.

8. A method of increasing feed conversion and growth-rate of suckling ruminants and poultry respectively, wherein solutions of ionophore antibiotics in water soluble non-toxic organic solvents incorporated in liquid feeds, milk replacers, water soluble vitamin concentrates or drinking water, as claimed in any one of claims 1 or 2, are administered to the animals.

9. A method of increasing feed conversion and rate of growth of suckling ruminants and poultry respectively, wherein solutions of ionophore antibiotics in water-soluble non-toxic organic solvents incorporated in liquid feeds, milk replacers, water soluble vitamin concentrates or in drinking water obtained by a process claimed in any one of claims 3 to 6 are administered to the animals.

10. Liquid milk replacers, liquid feeds, water soluble feeds, water soluble vitamin concentrates for ruminants, and drinking water for same and for poultry, admixed with ionophore antibiotics in water soluble non-toxic organic solvents whenever prepared according to a process claimed in any one of claims 3 to 6.